Europäisches Patentamt

**European Patent Office**

Office européen des brevets

(11) Veröffentlichungsnummer: **0 047 860
B1**

(12) **EUROPÄISCHE PATENTSCHRIFT**

(45) Veröffentlichungstag der Patentschrift:
27.12.84

(21) Anmeldenummer: **81106235.5**

(22) Anmeldetag: **10.08.81**

(51) Int. Cl.³: **G 01 N 33/00**

(54) Verfahren und Vorrichtung zur kalibrierten Dotierung eines Trägergases mit geringen Konzentrationen eines aggressiven Gases.

(30) Priorität: **08.09.80 DE 3033734**

(43) Veröffentlichungstag der Anmeldung:
**24.03.82 Patentblatt 82/12**

(45) Bekanntmachung des Hinweises auf die Patenterteilung:
**27.12.84 Patentblatt 84/52**

(84) Benannte Vertragsstaaten:
**AT BE CH DE FR GB IT LI LU NL SE**

(56) Entgegenhaltungen:
**EP - A - 0 012 617
US - A - 3 854 319
US - A - 4 056 966**

(73) Patentinhaber: **INTERATOM Internationale
Atomreaktorbau GmbH, Friedrich-Ebert-Strasse,
D-5060 Bergisch Gladbach 1 (DE)**

(72) Erfinder: **Hanebeck, Norbert, Dr., Kielsberg 50,
D-5063 Overath (DE)**

(74) Vertreter: **Mehl, Ernst, Dipl.-Ing. et al, Postfach 22 01 76,
D-8000 München 22 (DE)**

ACTORUM AG

## Beschreibung

Die vorliegende Erfindung betrifft ein Verfahren und eine Vorrichtung zur kalibrierten Dotierung eines Trägergases mit geringen Konzentrationen eines aggressiven Gases, insbesondere Fluorwasserstoff (chemische Formel: HF) oder Uranhexafluorid (chemische Formel: $UF_6$), nach dem Sättigungsdampfdruckprinzip. Unter geringen Konzentrationen, hier auch als vpm-Bereich bezeichnet, versteht man Volumenanteile der Beimischung von der Grössenordnung 1:1 000 000.

Aus der US-A-3 854 319 ist schon ein Verfahren zur Herstellung bestimmter Gas-Alkohol-Konzentrationen bekannt, bei welchem der Alkohol durch Oberflächenkräfte (Physisorption) an ein feinmaschiges Trägermaterial gebunden ist und von dort in das Trägergas freigegeben wird. Dieses Verfahren liefert zwar bei konstanten Betriebsbedingungen bestimmte Alkohol-Konzentrationen, jedoch stellt sich über dem mit Alkohol getränkten Trägermaterial kein nur exakt von der Temperatur abhängiger Partialdampfdruck ein. Für geringe Konzentrationen aggressiver Gase und stark veränderlicher Betriebsbedingungen ist das Gerät nicht geeignet.

Da es jedoch in vielen Bereichen der Technik, so z.B. bei Urananreicherungsanlagen, Müllverbrennungsanlagen oder auch im Bereich der chemischen Industrie, notwendig ist, die Konzentration von aggressiven Gasen, wie beispielsweise HF, im vpm-Bereich zu messen, ergibt sich die Notwendigkeit einer Kalibrierung der dabei verwendeten Messgeräte. Die auftretenden Probleme konnten für Spurenmessgeräte bisher nicht befriedigend gelöst werden, da zur Kalibrierung meist höhere Konzentrationen verwendet wurden, die dann eine Extrapolation in den vpm-Bereich verlangen.

Während in vielen Fällen, in denen es um die Kalibrierung von Gasspurenmessgeräten geht, hierfür handelsübliche Kalibriergase eingesetzt werden können, führt, wie noch gezeigt wird, ein ebensolcher Weg bei stark polaren Gasen wie HF nicht zum Ziel.

Da generell genügend stabile HF-Kalibriergasgemische für den vpm-Bereich nicht erhältlich sind, ist man darauf angewiesen, während der Gerätekalibrierung das HF-Gasgemisch kontinuierlich und reproduzierbar mit definierter Konzentration in einer geeigneten Apparatur herzustellen. Die einfachste Methode einer Gemischherstellung besteht darin, über Druckmessung eine bestimmte HF-Menge in einen Behälter einzulassen und anschliessend mit dem Trägergas auf den gewünschten Totaldruck aufzufüllen. Aufgrund der Wandadsorptionseigenschaft von HF würden sich beim ersten Schritt die Behälterwände bis zu einer Sättigung belegen und bei Eingabe des Zusatzgases eine Desorption von den Wänden erfolgen. Die wahre Verunreinigungskonzentration im Gas wäre somit nicht bekannt.

Würde man demgegenüber das Trägergas oberhalb eines HF-Reservoirs bestimmter Temperatur sättigen, kommt es besonders dann zu Schwierigkeiten, wenn grössere Gasmengen definierter Verunreinigungskonzentration erforderlich sind. Des weiteren ist eine homogene Temperaturverteilung im z.B. festen HF nur selten gewährleistet; ausserdem ist wegen der meist nur kleinen Oberfläche des festen HF aufgrund der verbrauchten Verdampfungswärme mit Temperaturerniedrigungen zu rechnen. Die zum jeweiligen Sättigungsdampfdruck gehörige Temperatur ist somit nicht mehr definiert.

Aufgabe der vorliegenden Erfindung sind ein Verfahren und eine Vorrichtung zur kalibrierten Dotierung eines Trägergases mit geringen Konzentrationen (vpm-Bereich) eines aggressiven Gases, insbesondere von HF und bei Verwendung desselben Adsorbers auch $UF_6$.

Diese Aufgabe wird dadurch gelöst, dass man die Temperaturabhängigkeit des Dissoziationsdrucks des zu kalibrierenden Gases über einem chemischen Adsorber für das entsprechende Gas ausnutzt. Die Chemiesorption steht, anders als die oben erwähnte Physisorption, im Reaktionsgleichgewicht mit dem partiellen Dampfdruck des jeweiligen Gases, wobei sich das Gleichgewicht in Abhängigkeit von der Temperatur ändert. Um eine schnelle Einstellung des jeweiligen Gleichgewichts zu erreichen, muss der Adsorber eine möglichst grosse Oberfläche besitzen. Ist er ausserdem im Gegensatz zur abgegebenen Gasmenge im grossen Überschuss vorhanden, sind Temperaturänderungen infolge Desorption vernachlässigbar.

Die einfachste technische Realisierung der kalibrierten Dotierung bestünde hiermit darin, den mit dem Gas beladenen Adsorber bei konstanter Temperatur innerhalb des Trägergasvolumens anzuordnen und die Einstellung des Gleichgewichts abzuwarten. Nach den bisherigen Versuchen führt diese Methode besonders im Bereich niedriger Partialdrücke aber nicht immer zum Ziel. Hierbei fungieren nämlich nach einiger Zeit die Behälterwände selbst als Adsorber, wobei deren «Dissoziationsdruck» den des Adsorbers dann übersteigen kann.

In weiterer Ausgestaltung der Erfindung wird daher im zweiten Anspruch vorgeschlagen, die Dotierung nicht statisch, sondern dynamisch erfolgen zu lassen, da dann eine weitgehende Unterdrückung der Wandeffekte im Hinblick auf die Einstellung definierter Verunreinigungsgrade im Trägergas gelingt. Versuche haben gezeigt, dass sich eine gut definierte Dotierung erreichen lässt, wenn ein Trägergasstrom definierter Temperatur und definierten Drucks kontinuierlich über den chemischen Adsorber geleitet wird.

In weiterer Ausgestaltung der Erfindung sieht der dritte Anspruch vor, das erfindungsgemässe Verfahren besonders zur Herstellung von HF- oder $UF_6$-Gemischen zu verwenden, da die Kalibrierung dieser Gase für Verunreinigungen im vpm-Bereich bisher am unvollkommensten gelöst ist.

In spezieller Ausgestaltung für diese Fälle wird im vierten Anspruch vorgeschlagen, als Adsorber Natriumfluorid (chemische Formel: NaF) zu verwenden, welches HF in der Form $NaF \cdot HF$ und $UF_6$ in den Formen $2NaF \cdot UF_6$ oder $3NaF \cdot UF_6$ bindet. Nach eingehender Untersuchung erscheint dieses Material als Adsorber am besten geeignet, erstens weil die Temperaturabhängigkeit des Dissoziationsdruckes der genannten Komplexe in der Literatur und aus eigenen Messungen hinreichend genau bekannt ist, und zweitens, weil die für die gewünschte Konzentration nötigen Temperaturen relativ leicht erreichbar sind.

Zur weiteren speziellen Ausgestaltung gemäss Anspruch 5 wird vorgeschlagen, das NaF als Granulat zu verwenden, da sich auf diese Weise die Anforderungen an die Oberfläche am besten erfüllen lassen.

Der Anspruch 6 schlägt als zusätzliche Ausgestaltung der Erfindung vor, das NaF-Granulat vor der Durchleitung des Trägergasstromes unter Luft- und Feuchteabschluss nur bis zu einem bestimmten Grad mit HF oder $UF_6$ zu beladen, da einerseits die Genauigkeit der Kalibrierung bei zu stark beladenem NaF nachlässt und andererseits die beim Betrieb freigesetzten Gasmengen so gering sind, dass auch bei geringer Beladung eine einzige Beschickung des Adsorbers über Monate ausreicht. Die Beladung sollte z.B. für HF unterhalb von 0,4 g HF/g NaF liegen.

Im Anspruch 7 wird eine Vorrichtung zur Durchführung des erfindungsgemässen Verfahrens angegeben. Dazu wird vorgeschlagen, einen Behälter für den chemischen Adsorber in einem thermostatisierbaren Behälter anzuordnen. Dadurch kann der chemische Adsorber auf eine exakt bekannte Temperatur oberhalb oder unterhalb der Umgebungstemperatur gebracht werden, was für die Genauigkeit der Eichung von entscheidender Bedeutung ist.

Darüberhinaus sieht eine weitere Ausgestaltung der Erfindung nach Anspruch 8 vor, das Trägergas durch ein gewendeltes Kupferrohr, welches in demselben thermostatisierbaren Behälter untergebracht ist, in den Behälter mit dem chemischen Adsorber zu leiten. Dadurch wird erreicht, dass das Trägergas schon die gleiche, genau definierte Temperatur wie der Adsorber hat, bevor es in den Behälter strömt.

Eine vollständige Sättigung des Trägergases erreicht man besonders leicht, wenn man in weiterer Ausgestaltung der Erfindung gemäss den Ansprüchen 9 und 10 in dem Behälter eine waagerechte Siebplatte befestigt, auf der der Adsorber aufgeschüttet ist und ein Tauchrohr unter die Siebplatte führt, durch das das Trägergas eingeleitet wird.

Als vorteilhaft bei der Eichung von Spurenmessgeräten zur Auffindung des Nullpunktes erweist sich auch die Ausgestaltung der Erfindung gemäss Anspruch 11. Dadurch, dass Aus- und Einlass des Behälters durch eine Umgehungsleitung mit Absperrventil verbunden sind, kann das Trägergas unter Umgehung des Behälters mit dem Adsorber weitergeleitet werden, so dass sich die einfache Möglichkeit, Reingas in das zu kalibrierende Gerät einzuleiten, ergibt.

Als zusätzliche Ausgestaltung wird vorgeschlagen, einen Temperaturfühler in den Raum für den chemischen Adsorber eintauchen zu lassen, um die genaue Temperatur zu bestimmen.

Ein Ausführungsbeispiel der Erfindung ist in der Zeichnung dargestellt und zwar zeigt

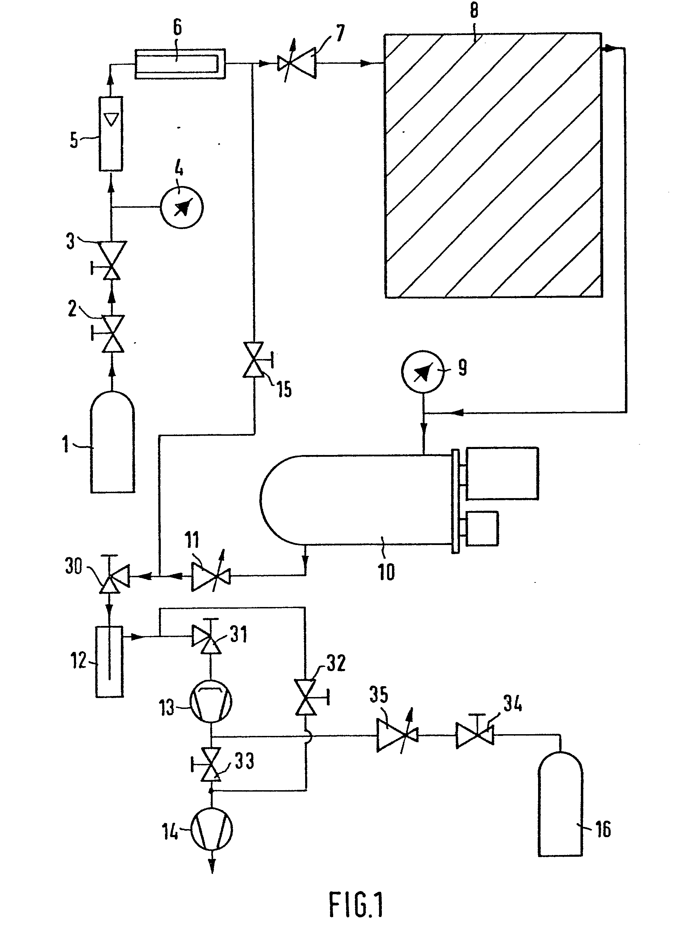

Fig. 1 schematisch eine Anordnung zur Kalibrierung eines HF-Spurenmessgerätes,

Fig. 2 schematisch den Aufbau der eigentlichen Dotiervorrichtung aus Fig. 1,

Fig. 3 ein Diagramm mit Ergebnissen, die bei der Eichung eines IR-Absorptionsspurenmessgerätes mit dieser Anordnung gewonnen wurden.

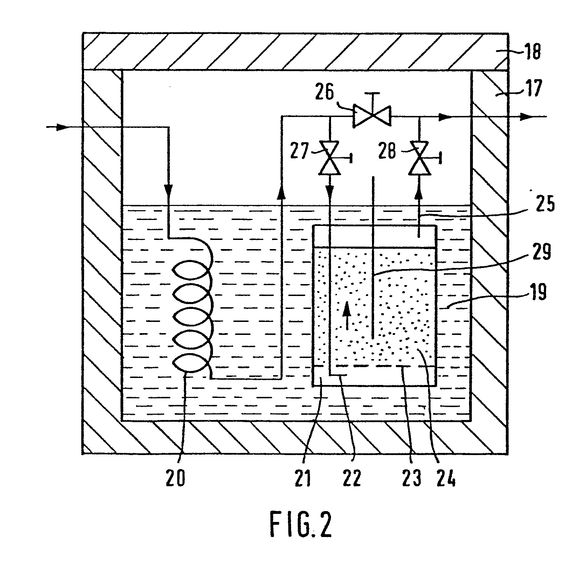

Das Trägergas gelangt aus einer Druckflasche 1 über ein Ventil 2 und einen Druckminderer 3, hinter dem der Druck an einem Druckmesser 4 abgelesen werden kann, in einen Durchflussmesser 5. Danach wird es durch einen Trockner 6 und ein Feindosierventil 7 in die eigentliche Dotiervorrichtung 8 (genauer beschrieben in Fig. 2) geleitet. Während vor dem Ventil 7 ein Druck von 1 bar aufrechterhalten wird (der Durchflussmesser 5 ist gerade für diesen Vordruck kalibriert), erfolgt hinter demselben die Druckabsenkung auf Werte, wie sie in der weiteren Anlage üblich sind. Nach der Dotierung gelangt das Kalibriergasgemisch über einen Druckmesser 9 in den zu kalibrierenden Analysator 10.

Am Feindosierventil 11 liegt das Pumpsystem, bestehend aus einer Kühlfalle 12, einer Diffusionspumpe 13 und einer Vorpumpe 14, mit voller Saugleistung an. Der Durchsatz am Ventil 11 wird gerade so geregelt, dass sich der gewünschte Anlagenbetriebsdruck – gemessen am Druckmesser 9 – einstellt.

Eine Leitung mit Absperrventil 15 ist zur Evakuierung zu Beginn und bei Beendigung der Versuche vorgesehen. Für den Fall, dass als Trägergas $H_2$ eingesetzt wird, kann die Vorpumpe aus Sicherheitsgründen zunächst mit Inertgas (z.B. $N_2$) aus einem Vorratsbehälter 16 gespült werden.

Die eigentliche Dotiervorrichtung 8, in Fig. 2 nochmals im Detail gezeigt, besteht aus einem thermostatisierbaren Behälter 17 mit Deckel 18, in dem sich ein für den vorgesehenen Temperaturbereich geeignetes Flüssigkeitsbad 19 befindet. Das Trägergas gelangt durch eine Leitung in den Behälter 17, wird dort in einer gewendelten Kupferrohrleitung 20 auf die Temperatur des Flüssigkeitsbades gebracht und gelangt dann zu einem ebenfalls in dem Behälter 17 untergebrachten Behälter 21. Über ein Tauchrohr 22 wird das Trägergas dann unter eine in dem Behälter waagerecht befestigte Siebplatte 23 geleitet, auf der NaF-Granulat 24 (mit HF beladen) aufgeschüttet ist. Das Trägergas strömt nun durch das HF-Granulat 24 zum Ausgang 25 und wird dabei mit HF gesättigt. Das so entstandene Kalibriergasgemisch wird dann wie oben beschrieben weitergeleitet. Eine Umgehungsleitung mit einem Absperrventil 26 zwischen Ein- und Auslass des Behälters in Verbindung mit zwei Ventilen 27, 28 ermöglicht eine Umgehung des Behälters, wodurch die einfache Möglichkeit besteht, reines Trägergas zum Analysator zu leiten, was zur Nullpunktbestimmung des zu kalibrierenden Messgerätes nötig ist.

Ein in den Raum für das NaF geführter Temperaturfühler 29 ermöglicht die genaue Feststellung der Temperatur.

Die Ventile 30–34 dienen zur Absperrung der verschiedenen Teile des Pumpsystems bzw. des Inertgasbehälters, das Ventil 35 zu Dosierung des Inertgases.

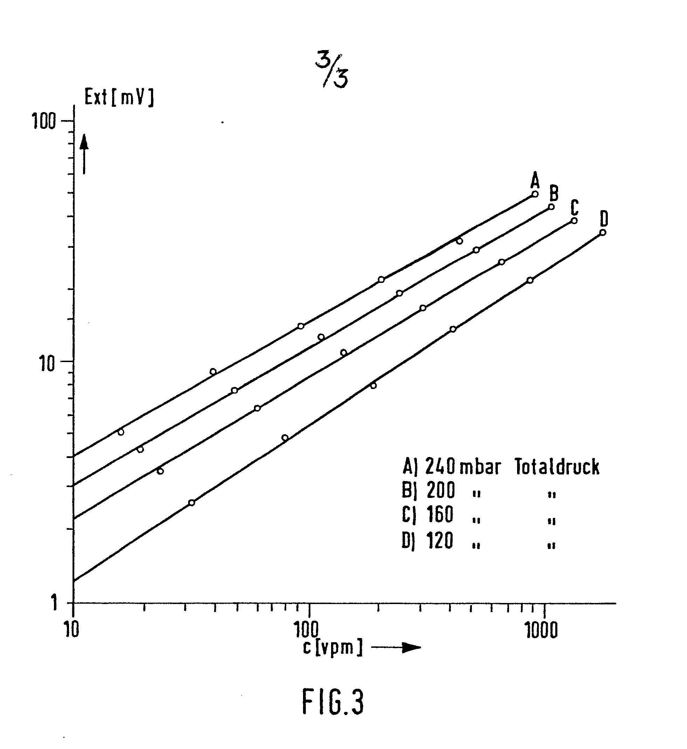

Die Fig. 3 zeigt im Diagramm einige Ergebnisse, die mit dem erfindungsgemässen Verfahren bei Testversuchen an einem Infrarot-Analysator erzielt wurden. Dort ist jeweils in logarithmischem Massstab die erzielte Infrarot-Absorption gegen die HF-Beimischung aufgetragen und zwar für verschiedene Systemdrücke.

Aus der Linearität der Eichgeraden im Bereich eines HF-Anteils von 10 bis $10^3$ vpm ist ersichtlich, dass sich das erfindungsgemässe Verfahren in diesem Bereich und nach beiden Seiten darüberhinaus zur Herstellung von Kalibriergasgemischen eignet, wobei auch noch der Druck des Kalibriergasgemisches in weiten Grenzen variabel ist. Wie aus der geringen Streuung der einzelnen Messpunkte um die jeweiligen Eichgeraden zu ersehen ist, kann die Kalibrierung mit Hilfe des erfindungsgemässen Verfahrens mit grosser Genauigkeit durchgeführt werden. Darüberhinaus ergaben sich bei Trägergas-Durchsatzänderungen bis zu einem Faktor 8 keine Änderungen der Messanzeige, was ein Beweis für die vollständige Sättigung des Trägergases mit HF ist.

Die erfolgreiche Anwendung der vorliegenden Erfindung bei der Herstellung von HF-Kalibriergasgemischen lässt erwarten, dass das erfindungsgemässe Verfahren auch zur Herstellung anderer Kalibriergasgemische, insbesondere von Wasserstoffhalogeniden, benutzt werden kann. Desweiteren konnte die Herstellung von $UF_6$-Kalibriergasgemischen experimentell nachgewiesen werden.

Zum Schluss sei noch die für das Ausführungsbeispiel nötige Eichung des Sättigungsdampfdruckes von HF über dem chemischen Adsorber NaF angegeben. Für den Dissoziationsdruck gilt folgende Beziehung:

$$\log p = 9,6000 - \frac{3,521 \cdot 10^3}{T} [\text{mbar}]$$

Im Bereich 0 bis 100 °C errechnet man danach folgende HF-Dampfdrücke:

| T[°C] | p [mbar] |
|---|---|
| 0 | $5,04 \cdot 10^{-4}$ |
| 10 | $1,44 \cdot 10^{-3}$ |
| 20 | $3,83 \cdot 10^{-3}$ |
| 30 | $9,54 \cdot 10^{-3}$ |
| 40 | $2,24 \cdot 10^{-2}$ |
| 50 | $5,00 \cdot 10^{-2}$ |
| 60 | $1,06 \cdot 10^{-1}$ |
| 70 | $2,16 \cdot 10^{-1}$ |
| 80 | $4,22 \cdot 10^{-1}$ |
| 90 | $7,95 \cdot 10^{-1}$ |
| 100 | $1,45 \cdot 10^{-1}$ |

Hieraus ist zu ersehen, dass NaF aufgrund des besonders günstigen Temperaturbereiches, in dem die gewünschten Konzentrationen hergestellt werden können, ein gut geeigneter Adsorber für die Herstellung von HF-Kalibriergasgemischen mit dem erfindungsgemässen Verfahren ist.

$UF_6$ bildet mit NaF die Komplexe $2NaF \cdot UF_6$ oder $3NaF \cdot UF_6$. Daraus resultieren zwei Dissoziationsdruckkurven, welche jedoch nahe benachbart liegen. $2NaF \cdot UF_6$ ist der Komplex mit dem höheren Dissoziationsdruck. Für ihn gilt folgende Beziehung:

$$\log p = 9,3740 - \frac{4,180 \cdot 10^3}{T} [\text{mbar}]$$

Dies bedeutet, dass gegenüber HF zur Einstellung identischer Spurenkonzentrationen höhere Sorbenstemperaturen anzuwenden sind.

Da völlig HF-freies $UF_6$ wegen der starken Affinität des letzteren gegenüber Feuchte nicht existiert, muss bei $UF_6$-Beladung des Adsorbers auch mit einer – wenn auch geringen – HF-Beladung desselben gerechnet werden. Die Partialdrücke beider stellen sich jedoch in Abhängigkeit von der Temperatur unabhängig voneinander ein. Insofern ist die Kalibrierung eines selektiven $UF_6$-Spurenmessgerätes auch in Anwesenheit von HF fehlerfrei möglich.

**Patentansprüche**

1. Verfahren zur kalibrierten Dotierung eines Trägergases mit geringen Konzentrationen eines aggressiven Gases nach dem Sättigungsdampfdruckprinzip, dadurch gekennzeichnet, dass die Temperaturabhängigkeit des Dissoziationsdrucks des zu kalibrierenden Gases über einem chemischen Adsorber für das entsprechende Gas ausgenutzt wird.

2. Verfahren nach Anspruch 1, dadurch gekennzeichnet, dass die Dotierung dynamisch erfolgt, indem ein Trägergasstrom definierter Temperatur und definierten Drucks kontinuierlich über den mit dem zu kalibrierenden Gas beladenen chemischen Adsorber geleitet wird.

3. Anwendung des Verfahrens nach Anspruch 1 oder 2 zur kalibrierten Dotierung eines Trägergases mit Fluorwasserstoff (HF) oder Uranhexafluorid ($UF_6$).

4. Verfahren nach Anspruch 1 oder 2 zur kalibrierten Dotierung eines Trägergases mit HF oder $UF_6$, dadurch gekennzeichnet, dass als Adsorber Natriumfluorid (NaF) verwendet wird.

5. Verfahren nach Anspruch 4, dadurch gekennzeichnet, dass das NaF als feinporiges Granulat vorliegt.

6. Verfahren nach Anspruch 5, dadurch gekennzeichnet, dass das NaF-Granulat vor der Durchleitung des Trägergasstromes unter Luft- und Feuchteabschluss bis zu einem bestimmten Grad mit HF oder $UF_6$ beladen wird.

7. Vorrichtung zur kalibrierten Dotierung eines Trägergases mit geringen Konzentrationen eines aggressiven Gases nach dem Sättigungsdampfdruckprinzip, entsprechend dem Verfahren nach Anspruch 2, dadurch gekennzeichnet, dass in einem thermostatisierbaren Behälter (17) ein von einem Trägergas durchströmbarer Behälter (21) für einen mit dem aggressiven Gas beladbaren chemischen Adsorber (24) angeordnet ist.

8. Vorrichtung nach Anspruch 7, dadurch gekennzeichnet, dass ein gewendeltes Kupferrohr (20) als Zuleitung für das Trägergas in dem thermostatisierbaren Behälter (17) untergebracht ist und zu dem Behälter (21) mit dem Adsorber führt.

9. Vorrichtung nach Anspruch 7 oder 8, dadurch gekennzeichnet, dass in dem Behälter (21) eine waagerechte Siebplatte (23) für den Adsorber befestigt ist.

10. Vorrichtung nach Anspruch 9, dadurch gekennzeichnet, dass ein Tauchrohr (22) für das Trägergas unter die Siebplatte (23) führt.

11. Vorrichtung nach Anspruch 7, 8, 9 oder 10, dadurch gekennzeichnet, dass Ein- und Auslass des Behälters (21) durch eine Umgehungsleitung mit Absperrventil (26) verbunden sind.

12. Vorrichtung nach einem der Ansprüche 7 bis 11, dadurch gekennzeichnet, dass ein Temperaturfühler (29) in dem Raum für den chemischen Adsorber (24) vorhanden ist.

## Revendications

1. Procédé de dopage calibré d'un gaz porteur par de faibles concentrations d'un gaz agressif suivant le principe de la tension de vapeur saturante, caractérisé en ce qu'il consiste à tirer parti de la variation, en fonction de la température, de la pression de dissociation du gaz à calibrer sur un adsorbant chimique pour le gaz correspondant.

2. Procédé suivant la revendication 1, caractérisé en ce qu'il consiste à effectuer le dopage d'une manière dynamique en envoyant un courant de gaz porteur ayant une température définie et une pression définie en continu sur l'adsorbant chimique chargé du gaz à calibrer.

3. Utilisation du procédé suivant la revendication 1 ou 2 pour le dopage calibré d'un gaz porteur par de l'acide fluorydrique (HF) ou de l'hexafluorure d'uranium (UF$_6$).

4. Procédé suivant la revendication 1 ou 2 de dopage calibré d'un gaz porteur par HF ou par UF$_6$, caractérisé en ce qu'il consiste à utiliser comme adsorbant du fluorure de sodium (NaF).

5. Procédé suivant la revendication 4, caractérisé en ce que le NaF se présente sous la forme d'un produit en grains à pores fins.

6. Procédé suivant la revendication 5, caractérisé en ce qu'il consiste à charger de HF ou de UF$_6$ jusqu'à un taux déterminé en opérant à l'abri de l'air et de l'humidité le produit en grains en NaF avant que le courant de gaz porteur ne le traverse.

7. Dispositif de dopage calibré d'un gaz porteur par de faibles concentrations d'un gaz agressif suivant le principe de la tension de vapeur saturante conformément au procédé suivant la revendication 2, caractérisé en ce que dans un récipient (17) pouvant être thermostatisé est disposé un récipient (21) qui peut être traversé par un gaz porteur et qui contient un adsorbant chimique (24) pouvant être chargé par le gaz agressif.

8. Dispositif suivant la revendication 7, caractérisé en ce qu'un serpentin tubulaire en cuivre (20) servant de conduit d'amenée au gaz porteur est logé dans le récipient (17) pouvant être thermostatisé et conduit au récipient (21) ayant l'adsorbant.

9. Dispositif suivant la revendication 7 ou 8, caractérisé en ce que dans le récipient (21) est fixée une plaque perforée horizontale (23) pour l'adsorbant.

10. Dispositif suivant la revendication 9, caractérisé en ce qu'un tube plongeur (22) pour le gaz porteur débouche sous la plaque perforée (23).

11. Dispositif suivant la revendication 7, 8, 9 ou 10, caractérisé en ce que l'entrée et la sortie du récipient (21) communiquent par un conduit de dérivation ayant un robinet d'arrêt (26).

12. Dispositif suivant l'une des revendications 7 à 11, caractérisé en ce qu'une sonde de température (29) est présente dans la chambre pour l'adsorbant chimique (24).

## Claims

1. A process for the measured addition to a carrier gas of small concentrations of a corrosive gas in accordance with the saturation vapour pressure principle, characterised in that the temperature dependency of the dissociation pressure of the gas to be measured, is made use of by means of a chemical adsorber for the gas.

2. A process according to Claim 1, characterised in that the measured addition is effected dynamically, a carrier gas stream at a definite temperature and definite pressure being continuously passed over the chemical adsorber which is charged with the gas to be measured.

3. The use of the process according to Claim 1 or 2 for the measured addition to a carrier gas of hydrogen fluoride (HF), or uranium hexafluoride (UF$_6$).

4. A process according to Claim 1 or 2 for the measured addition to a carrier gas of HF or UF$_6$, characterised in that sodium fluoride (NaF) is used as adsorber.

5. A process according to Claim 4, characterised in that the NaF is present as finely porous granulate.

6. A process according to Claim 5, characterised in that, before the carrier gas stream is passed through, the NaF-granulate is charged with HF or UF$_6$ with the exclusion of air and moisture to a predetermined degree.

7. Apparatus for the measured addition to a carrier gas of small concentrations of a corrosive gas in accordance with the saturation vapour pressure principle according to the process as claimed in Claim 2, characterised in that in a container (17), which can be thermostatically controlled, there is arranged a container (21), through which a carrier gas can flow, for a chemical adsorber (24) which can be charged with a corrosive gas.

8. Apparatus according to Claim 7, characterised in that a coiled copper tube (20) is housed in the container (17), which can be thermostatically controlled, as a supply line for the carrier gas and leads to the container (21) having the adsorber.

9. Apparatus according to Claim 7 or 8, characterised in that a horizontal perforated plate (23) for the adsorber is fastened in the container (21).

10. Apparatus according to Claim 9, characterised in that a dip tube (22) for the carrier gas leads below the perforated plate (23).

11. Apparatus according to Claim 7, 8, 9 or 10, characterised in that the inlet and the outlet of the container (21) are connected by a by-pass line with a stop valve (26).

12. Apparatus according to one of Claims 7 to 11, characterised in that a temperature sensor (29) is located in the space for the chemical adsorber (24).

1/3

FIG.1

2/3

FIG.2

3/3

FIG.3